# FASCICULE DE BREVET EUROPEEN

(11) **EP 2 521 767 B1**
(45) Date de publication et mention de la délivrance du brevet: **18.03.2015**
(21) Numéro de dépôt: 11700519.9
(22) Date de dépôt: 04.01.2011
(51) Int. Cl.: C12M 1/00

(54) **PHOTOBIOREACTEUR EN MILIEU FERME POUR LA CULTURE DE MICRO-ORGANISMES PHOTOSYNTHETIQUES**
FOTOBIOREKATOR IN EINER GESCHLOSSENEN UMGEBUNG ZUR KULTIVIERUNG VON FOTOSYNTHETISCHEN MIKROORGANISMEN
PHOTOBIOREACTOR IN A CLOSED ENVIRONMENT FOR CULTIVATING PHOTOSYNTHETIC MICRO-ORGANISMS

(30) Priorité: 04.01.2010 FR 1050015
(43) Date de publication de la demande: 14.11.2012
(73) Titulaire: Acta Alga, 75017 Paris (FR)
(72) Inventeur: BOURGOIN, Jacques, F-77810 Thomery (FR); CONIN, Michel, F-75116 Paris (FR); FRIEDERICH, Alain, F-75007 Paris (FR); SUN, Guocai, F-92340 Bourg La Reine (FR)
(74) Mandataire: Regimbeau
(86) Numéro de dépôt international: PCT/EP2011/050050
(87) Numéro de publication internationale: WO 2011/080345

(56) Documents cités:
- WO-A2-2005/101525
- WO-A2-2009/069967
- DE-A1-102007 000 815
- US-A1- 2009 246 863

## Description

L'invention concerne la culture intensive et continue de micro-organismes photosynthétiques.

Les microalgues sont des organismes végétaux photosynthétiques dont le métabolisme et la croissance nécessitent entre autres du CO₂, de la lumière et des nutriments.

La culture industrielle de microalgues connaît de nombreuses applications.

Les microalgues peuvent être cultivées pour valoriser et purifier les rejets en dioxyde de carbone, NOx et/ou SOx de certaines usines (WO 2008042919).

L'huile extraite des microalgues peut être utilisée comme biocarburant (WO2008070281, WO2008055190, WO2008060571).

Les microalgues peuvent être cultivées pour leur production d'oméga-3 et d'acides gras polyinsaturés.

Les microalgues peuvent également être cultivées pour produire des pigments.

La culture industrielle de microalgues à grande échelle utilise le soleil comme source de lumière. Pour ce faire, les microalgues sont souvent placées dans des bassins ouverts (« raceways ») avec ou sans circulation (US2008178739). On trouve également des photobioréacteurs tubulaires ou à plaques, constitués de matériaux translucides, permettant le passage des rayons lumineux dans le milieu de culture et dans lesquels les microalgues circulent (FR2621323). D'autres systèmes de réseaux de tubes transparents en trois dimensions permettent d'améliorer l'exploitation de l'espace (EP0874043).

Ces installations sont extrêmement volumineuses et les rendements de production sont faibles étant donné les aléas d'éclairement du soleil et les phases de nuit néfastes à la croissance des microalgues.

Afin de réduire l'encombrement et d'améliorer le rendement, des photobioréacteurs fermés ont été mis au point. Ils utilisent quant à eux la disponibilité d'un éclairage artificiel 24h/24 et 7 jours/7, cet éclairage pouvant être interrompu suivant des séquences propres aux cycles biologiques des algues concernées.

En effet, le facteur crucial de l'augmentation de la biomasse des micro-algues est la lumière, aussi bien en termes de quantité que de qualité puisque les micro-algues absorbent uniquement certaines longueurs d'ondes de la lumière blanche.

Un photobioréacteur est défini comme un système clos à l'intérieur duquel se déroulent des interactions biologiques, en présence d'énergie lumineuse, que l'on cherche à contrôler en maîtrisant les conditions de culture.

Plus la lumière dispensée dans le photobioréacteur sera adaptée à l'espèce de micro-algues, plus la production de biomasse sera intéressante.

Une première solution d'éclairage artificiel pour résoudre ce problème consiste à amener la lumière d'une source lumineuse dans le milieu de culture à proximité des microalgues à l'aide de fibres optiques (US6156561 et EP0935991).

Les fibres optiques peuvent être associées à d'autres moyens immergés guidant la lumière à l'intérieur de l'enceinte (JP2001178443 et DE29819259).

L'inconvénient majeur est que cette solution ne permet d'atteindre que des rendements (lumière produite)/(lumière efficace) faibles. En effet, l'intensité est réduite du fait des interfaces entre les sources lumineuses et le guide d'onde et il est difficile de coupler plus d'une source lumineuse sur la même fibre. En outre, un problème se pose dès lors que l'on utilise plusieurs longueurs d'ondes différentes : En effet pour sortir la lumière des fibres optiques immergées dans le milieu de culture, il est nécessaire de faire un traitement surface (rugosité), qui diffusera ou diffractera une fraction de la lumière guidée. La solution la plus efficace consiste à graver un réseau à la périphérie de la fibre avec un pas qui est de l'ordre de la longueur d'onde de la lumière véhiculée. Cette solution a une bande passante étroite et est totalement inadaptée quand on utilise plusieurs longueurs d'ondes. L'usage d'une rugosité aléatoire quant à elle, est de faible efficacité.

Une autre solution d'éclairage artificiel pour résoudre ce problème consiste à immerger directement des sources lumineuses dans l'enceinte du photobioréacteur, comme par exemple des lampes fluorescentes (US 5,104,803) ou des LEDs (Light Emitting Diode) (DE202007013406 et WO2007047805).

Cette solution permet d'améliorer le rendement énergétique du procédé d'éclairage car les sources lumineuses sont plus proches et mieux couplées au milieu de culture.

Toutefois, l'utilisation de sources lumineuses introduites au sein du réacteur, en particulier des LEDs, doit se faire en tenant compte de trois autres problèmes majeurs.

Le premier est inhérent à la pénétration de la lumière dans la culture, laquelle est directement liée à la densité des microalgues. Cette densité croît au cours du processus de culture et conduit rapidement à l'extinction du flux lumineux dans la majeure partie du réacteur. Les solutions consistant à éclairer la paroi interne du photobioréacteur (DE202007013406) ne sont donc pas transposables à des photobioréacteurs d'échelle industrielle de plusieurs centaines de litres par simple homothétie, les longueurs d'absorption de la lumière étant toujours centimétriques en fin de processus d'élevage.

Pour supprimer les zones d'ombres apparaissant au cours du processus de culture, on peut multiplier les sources lumineuses dans l'enceinte et les implanter suffisamment proches les unes des autres pour éclairer le milieu de culture indépendamment des longueurs d'absorption variables liées au cycle biologique. Ce faisant se pose alors le problème de la gestion de la thermique du réacteur qui doit être contrôlée à quelques degrés près, et qui dépend de la nature de l'algue. Cette gestion de la thermique constitue le deuxième problème majeur qu'il est nécessaire de résoudre. Il est inhérent à ces structures de réacteurs de première génération, indépendamment du type de sources lumineuses utilisées. S'ajoute le problème du coût du photobioréacteur si les sources lumineuses doivent être multipliées en grand nombre.

Le troisième problème est d'obtenir un front d'éclairage homogène en intensité dans le volume de croissance du réacteur. A la décroissance progressive de l'intensité de l'onde lumineuse par absorption dans le milieu de culture vient s'ajouter une forte dispersion de l'énergie lumineuse sur le front lumineux incident. Ceci est préjudiciable à l'optimisation du procédé de croissance de biomasse pour une énergie incidente lumineuse globale donnée.

Afin de régler ces problèmes, les inventeurs ont découvert, de manière inattendue et surprenante, une nouvelle source de lumière adaptée aux photobioréacteurs : les cellules photovoltaïques utilisées en injection directe qui dans ces conditions émettent de la lumière.

Cette source de lumière présente les avantages d'être particulièrement homogène et de pouvoir être optimisée pour la souche d'algues à produire puisque les cellules photovoltaïques peuvent être adaptées pour émettre la ou les longueurs d'ondes absorbée(s) par la souche pour sa photosynthèse.

Par conséquent, l'objet de l'invention concerne un photobioréacteur destiné à la culture de micro-organismes photosynthétiques, de préférence de microalgues, comprenant :
(a) au moins une enceinte de culture (1) destinée à contenir le milieu de culture (3) des microorganismes,
(b) des cellules photovoltaïques (2) isolées du milieu de culture (3) émettant de la lumière vers le milieu de culture (3)
(c) des moyens d'alimentation électrique (4) des cellules photovoltaïques (2) afin de faire fonctionner les cellules photovoltaïques en mode d'émission de lumière.
Une cellule photovoltaïque est un composant électronique qui, exposé à la lumière (photons), génère de l'électricité. Les cellules photovoltaïques les plus répandues sont constituées de matériaux semi-conducteurs. Pour obtenir une émission de lumière, il est nécessaire que ces matériaux semi-conducteurs soient à gap direct, tels que les alliages As, Ga, In, P. Le matériau silicium (Si) est inadapté à cette fonction car son gap est indirect. Elles se présentent généralement sous la forme de fines plaques d'une dizaine de centimètres de côté, prises en sandwich entre deux contacts métalliques, pour une épaisseur de l'ordre du millimètre. Le principe des cellules photovoltaïques est bien connu (Physics of Semiconductor Devices-J Wiley & Sons, 3rd Edition, Simon M. Sze, Kwok K. Ng).

Dans le semi-conducteur exposé à la lumière, un photon d'énergie suffisante arrache un électron, créant au passage un "trou". Normalement, l'électron trouve rapidement un trou pour se replacer, et l'énergie apportée par le photon est ainsi dissipée. Le principe d'une cellule photovoltaïque est de forcer les électrons et les trous à se diriger chacun vers une face opposée du matériau au lieu de se recombiner simplement en son sein : ainsi, il apparaîtra une différence de potentiel et donc une tension entre les deux faces, comme une pile.

Pour cela, on s'arrange pour créer un champ électrique permanent au moyen d'une jonction PN, entre deux couches dopées respectivement P et N. Dans la couche supérieure de la cellule, il existe une quantité d'électrons libres supérieure à une couche de matériau pur, d'où l'appellation de dopage N, comme négatif (charge de l'électron).
Dans la couche inférieure de la cellule la quantité d'électrons libres est inférieure à celle d'une couche de matériau pur, les électrons sont liés au réseau cristallin qui, en conséquence, est chargé positivement. La conduction électrique est assurée par des trous, positifs (P).

Au moment de la création de la jonction P-N, les électrons libres de la région N rentrent dans la couche P et vont se recombiner avec les trous de la région P. Il existera ainsi, pendant toute la vie de la jonction, une charge *positive* de la région N au bord de la jonction (parce que les électrons en sont partis) et une charge *négative* dans la région P au bord de la jonction (parce que les trous en sont disparus) et il existe un champ électrique entre les deux, de N vers P.

En fonctionnement classique, un photon arrache un électron à la matrice, créant un électron libre et un trou. Les électrons s'accumulent dans la région N (qui devient le pôle négatif), tandis que les trous s'accumulent dans la couche dopée P (qui devient le pôle positif).

Des cellules ayant une grande efficacité ont été développées pour des applications spatiales : les cellules multi-jonctions qui sont constituées de plusieurs couches minces, classiquement de une à cinq jonctions.

Une cellule triple jonction, par exemple, est constituée des semi-conducteurs AsGa, Ge et GaInP2. Chaque type de semi-conducteur est caractérisé par une longueur d'onde maximale au delà de laquelle il est incapable de convertir le photon en énergie électrique. En deçà de cette longueur d'onde, le surplus d'énergie véhiculé par le photon est perdu.

Selon la présente invention, les cellules photovoltaïques sont utilisées en mode inverse d'émission c'est-à-dire comme source de lumière. Elles sont alimentées en courant électrique appelé « courant d'injection » et inversement à leur fonctionnement classique décrit ci-dessus produisent de la lumière. Si l'on applique une tension positive du côté de la région P, les porteurs majoritaires positifs (les trous) sont repoussés vers la jonction. Dans le même temps, les porteurs majoritaires négatifs du côté N (les électrons) sont attirés vers la jonction. Arrivés à la jonction, les porteurs se recombinent en libérant des photons ayant des énergies correspondant aux gaps des matériaux semi-conducteurs utilisés. Fondamentalement, une cellule photovoltaïque utilisée en injection directe est une diode électroluminescente de grande surface. Elle diffère de plus d'une LED par la géométrie de ses contacts d'injection qui doivent couvrir une grande surface. Classiquement, on réalise des réseaux de contacts avec des doigts espacés d'une longueur inférieure à la longueur de diffusion des porteurs. Cette LED grande surface peut bénéficier de toutes les améliorations des rendements quantiques interne et externe mises en oeuvre dans les LEDs classiques (réflecteur de Bragg, utilisation de puits quantiques dans la couche active, traitements de surface, etc). En effet, pour sortir du dispositif, les photons doivent traverser (sans être absorbés) le semi-conducteur, de la jonction jusqu'à la surface, puis traverser la surface du semi-conducteur sans subir de réflexion et, notamment, ne pas subir la réflexion totale interne qui renvoie les photons vers l'intérieur de la cellule où ils finissent par être absorbés. Ceux qui ne sont pas soumis à la réflexion totale interne sortent du semi-conducteur et constituent le flux optique externe (vers l'air par exemple).

Dans les LEDs ponctuelles, on améliore marginalement l'efficacité de transfert externe en introduisant une optique collée sur la surface du semiconducteur (indice optique intermédiaire entre celui de l'air (n=1) et celui du semiconducteur (3<n<4). Dans ces conditions, les meilleures LED ont des rendements quantiques externes proches de 20% (puissance lumineuse externe sur puissance électrique fournie au composant). Pour une LED plane de plus grande taille selon l'invention, la solution sera de microstructurer la surface de façon à augmenter la probabilité que le photon rencontre une surface quasi perpendiculairement. Le plus haut rendement quantique externe jamais obtenu, est pour le moment un peu supérieur à 45%. Divers procédés de microstructuration sont en cours d'étude en laboratoire, ils reposent sur des techniques de lithographie micronique en usage dans l'industrie des semiconducteurs, ou sur des techniques d'attaque chimique de la surface externe de la LED. Dans cette dernière classe de technologies, des rendements quantiques externes de l'ordre de 30% sont couramment obtenus. Le fait d'utiliser des composants de grandes surfaces rend l'application de ces technologies de micro-structuration beaucoup plus aisée.

Les cellules photovoltaïques utilisées selon la présente invention sont en matériau à gap direct (AsGa, GaInP, etc...). Dans ces matériaux, l'énergie libérée lors de la recombinaison d'une paire trou-électron se traduit par l'émission d'un photon éventuellement visible. L'intensité lumineuse est directement proportionnelle au courant d'injection. La longueur d'onde d'émission lumineuse correspond à l'énergie du gap du matériau semi-conducteur constituant la cellule photovoltaïque. Un matériau semi-conducteur à gap indirect n'émet pas de lumière, l'énergie se dissipant sous forme de chaleur. Classiquement, les matériaux à gap direct et émettant dans le visible sont des alliages III/V ou II/VI.

La lumière émise correspond à des transitions radiatives directes des matériaux constituants de la cellule photovoltaïque. Ainsi, on peut choisir une cellule photovoltaïque fabriquée en un ou plusieurs matériaux émettant dans une ou plusieurs longueurs d'ondes, avantageusement la ou les longueurs d'ondes de l'espèce de micro-organisme photosynthétique qui va être cultivé dans le photobioréacteur de l'invention.

Préférentiellement, les cellules photovoltaïques utilisées dans la présente invention sont des cellules à une, deux ou trois jonctions.

Préférentiellement, leur substrat est le germanium ou l'AsGa qui ont des paramètres de maille comparables à ceux des matériaux qu'on souhaite épitaxier pour réaliser les jonctions. L'usage du silicium en tant que substrat nécessite, comme il a été montré dans la littérature, d'utiliser la technologie dite Smart-Cut®. Cette dernière consiste à séparer la partie active du composant (réalisée sur une couche d'AsGa ou de Germanium) et à la coller par adhésion moléculaire sur le substrat de silicium. Préférentiellement, les matériaux à gap direct recouvrant le substrat sont des alliages III/V selon le tableau périodique des éléments, de manière particulièrement préférée l'AsGa (Arsenic-Gallium), le GaInP (Gallium-Indium-Phosphore) et/ou le GaInAs (Gallium-Indium-Arsenic), bien que tous les matériaux à gap direct soient appropriés.

De manière particulièrement préférée, les cellules photovoltaïques (2) utilisées dans la présente invention sont des cellules en matériau AsGa et/ou GaInP sur substrat germanium.

Les matériaux sont choisis en fonction de leur longueur d'ondes émettrice. En effet, un des avantages du photobioréacteur de l'invention est de fournir au micro-organisme photosynthétique cultivé la ou les longueurs d'ondes spécifiques qu'il absorbe pour sa photosynthèse et ainsi optimiser les conditions de multiplication de la biomasse.

Avantageusement, les cellules photovoltaïques utilisées selon l'invention présentent un substrat et un ou deux matériaux à gap direct, soit deux ou trois jonctions, et émettent à une ou deux longueurs d'onde. Avantageusement, elles émettent à des longueurs d'ondes correspondant aux pigments chlorophylliens. Avantageusement, elles émettent à des longueurs d'ondes comprises dans les intervalles 400 à 450 nm et 640 à 700 nm.

Les cellules photovoltaïques mesurent quelques dizaines de centimètres carré, classiquement environ 100 cm². Selon la présente invention, elles sont de préférence disposées sur des plaques (7). De manière particulièrement préférée, elles recouvrent des plaques (7) pour former par juxtaposition un système d'éclairement homogène plan jusqu'à une surface de l'ordre du mètre carré. Elles peuvent être disposées sur une ou les deux faces de la plaque. Elles peuvent être constituées de matériaux différents de chaque côté de la plaque. Par exemple, un côté de la plaque peut être couvert par des cellules photovoltaïques émettant une longueur d'ondes et l'autre côté de la plaque peut être couvert par des cellules photovoltaïques émettant une autre longueur d'ondes.

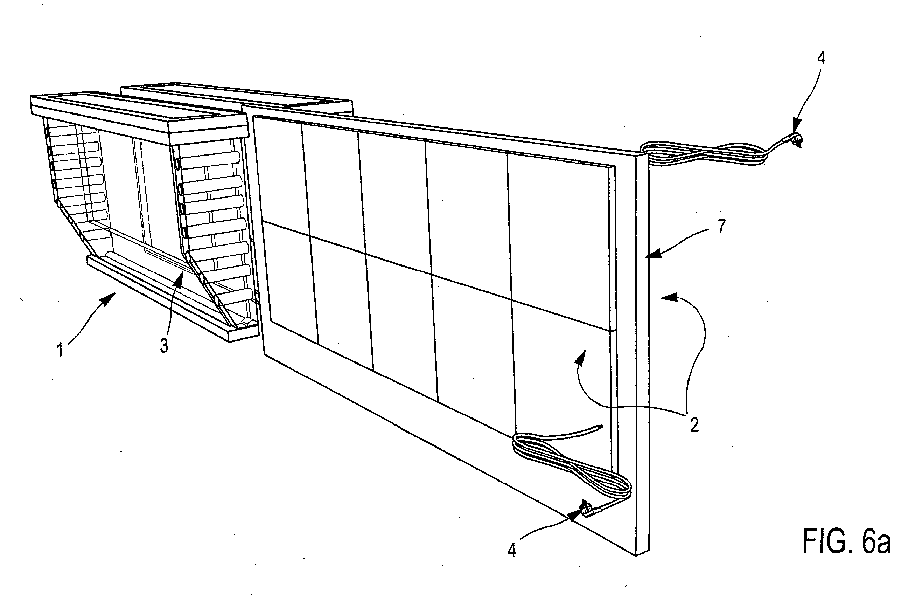

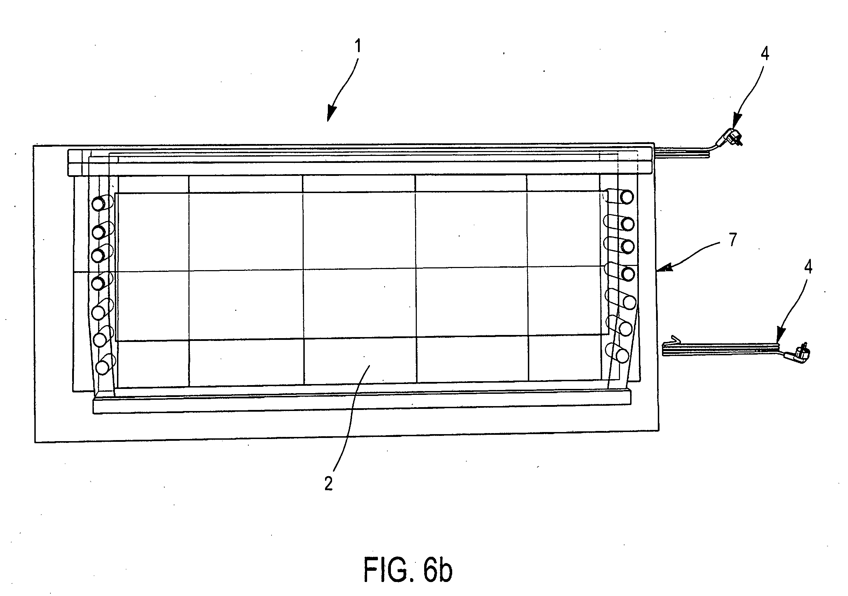

Les cellules photovoltaïques (2) sont de préférence placées :
- soit dans la ou les enceinte(s) de culture (1) dans des enceintes de transparence adaptée (TA) et étanches (5) immergées dans le milieu de culture (3),
- soit à l'extérieur de la ou des enceintes de culture, à faible distance de la paroi externe de la ou des enceinte(s) de culture (6), ladite paroi étant constituée d'un matériau de transparence adaptée au passage de la ou des longueurs d'ondes qu'elles émettent. Dans un mode de réalisation particulier, le photobioréacteur de l'invention comprend plusieurs enceintes de culture séparées par des cellules photovoltaïques. Par exemple, plusieurs enceintes de culture parallélépipédiques, par exemple deux, sont empilées et séparées par des plaques (7) de cellules photovoltaïques (2) (voir Figures 6).

Les enceintes de « transparence adaptée » (TA) sont des enceintes qui assurent un rendement optique optimum dans les longueurs d'ondes assurant la photosynthèse. Les matériaux de transparence adaptée appropriés sont le PMMA (polyméthacrylate de méthyle), le plexiglas, le verre, le polycarbonate, des plaques PMMA.

On entend par « faible distance » de quelques millimètres à quelques dizaines de centimètres, de préférence de quelques millimètres à quelques centimètres.

En particulier, il s'agit d'une distance de 0,1 à 20 cm, de manière préférée de 0,5 à 5 cm, de manière encore préférée de 0,5 à 2 cm, de manière particulièrement préférée d'environ 1 cm.

Dans un certain type de fonctionnement du photobioréacteur, on peut préférer utiliser une faible concentration de micro-algues. Ceci conduit à ce qu'une fraction significative de la lumière soit non absorbée par le milieu de culture et sorte donc du réacteur. On peut renvoyer cette lumière dans le milieu de culture pour un second passage, en transformant la paroi externe du réacteur en miroir (métallisation Al, Ag par exemple).

L'enceinte de culture (1) présente classiquement une forme cylindrique ou parallélépipédique.

Les cellules photovoltaïques sont alimentées en courant par le biais de contacts d'injection (8). Ces contacts (8) sont de préférence disposés à l'extrémité des plaques de cellules photovoltaïques (2) émettrices. Ils sont faiblement résistifs dits ohmiques. La modulation du pas du réseau des contacts permet de moduler spatialement le front de l'énergie lumineuse émise.

Préférentiellement, les cellules photovoltaïques sont assemblées selon une architecture série.

Avantageusement, les cellules photovoltaïques sont isolées électriquement de leur support par un isolant de bonne conductivité thermique, par exemple de type Mylar®, un film de polyéthylène téréphtalate développé par DuPont de Nemours.

Selon un mode de réalisation, le photobioréacteur de l'invention comprend un système de refroidissement (9) des cellules photovoltaïques (2). Avantageusement, le système de refroidissement (9) est constitué d'un fluide caloporteur (10) circulant dans les enceintes étanches (5), lesdites enceintes (5) étant reliées à un dispositif de refroidissement extérieur aux enceintes étanches pour le fluide caloporteur (10).

Avantageusement, le fluide caloporteur (10) est choisi pour sa transparence dans la gamme de longueurs d'ondes de 0,3 micron à 1 micron et il ne doit pas avoir d'absorption significative dans cette gamme de longueur d'ondes. Des fluides caloporteurs appropriés sont l'huile de silicone, l'huile perfluorée ou l'air.

Le fluide caloporteur (10) refroidit directement les cellules photovoltaïques (2) par contact. Il est lui-même dirigé vers et refroidi par le système de refroidissement du photobioréacteur de l'invention, externe à l'enceinte de culture (1). La thermorégulation de ce fluide permet en outre de thermostater l'enceinte de culture.

Le photobioréacteur de l'invention peut comprendre en outre un système d'injection de gaz (11), en particulier de CO₂ dans l'enceinte de culture (1).

L'enceinte de culture (1) du photobioréacteur selon l'invention peut être dimensionnée pour des applications industrielles variées ou de laboratoire.

Les dimensions d'une enceinte de culture (1) à l'échelle du laboratoire sont de quelques dizaines de centimètres à quelques centaines de centimètres pour la hauteur et le diamètre (enceinte cylindrique) ou la largeur (enceinte parallélépipédique). Le volume d'une enceinte de culture (1) à l'échelle du laboratoire est de moins de un m³. Avantageusement, l'enceinte de culture (1) est une enceinte de culture (1) industrielle.

Les dimensions d'une enceinte de culture (1) à l'échelle industrielle sont de plusieurs mètres.

Le volume d'une enceinte de culture (1) à l'échelle industrielle est supérieure à un m³ L'enceinte de culture (1) est réalisée en un matériau adapté pour contenir le milieu de culture, métallique ou en polymère par exemple, et, préférentiellement choisi dans le groupe constitué du PMMA, de polycarbonate ou de l'inox. On peut également prévoir des enceintes en matériau de construction type béton par exemple.

Selon le mode de réalisation où les cellules photovoltaïques sont placées à l'extérieur de l'enceinte de culture, l'enceinte de culture est réalisée en un matériau de transparence adaptée.

Selon le mode de réalisation où les cellules photovoltaïques sont placées dans l'enceinte de culture, les parois internes (12) de l'enceinte de culture (1) du photobioréacteur sont avantageusement réfléchissantes afin de réduire au maximum la perte de rayon lumineux en dehors de l'enceinte fermée. Elles peuvent être couvertes d'une peinture ou d'un matériau réfléchissant. Ainsi on réduit la dépense énergétique nécessaire à la culture des micro-organismes photosynthétiques.

Le photobioréacteur de l'invention peut comprendre en outre un système de brassage (13) du milieu de culture (3).

Le système de brassage (13) a deux fonctions principales. D'une part, il doit favoriser l'homogénéisation de la température du milieu de culture. D'autre part, il améliore l'homogénéisation de l'éclairement des microorganismes. En effet, grâce à ce brassage on fait passer les microorganismes des zones les plus éclairées aux zones les moins éclairées et inversement.

Le brassage du milieu de culture est réalisé par diverses techniques la plus courante actuellement est appelée « air-lift ». On peut aussi utiliser des types d'agitations mécaniques : vis d'Archimède, hélice .marine, de type Rushton, hydrofoil etc.

Avantageusement, la technique de brassage utilisée est celle appelée « air lift » qui consiste à injecter un gaz pressurisé, par exemple de l'air, dans la partie basse de l'enceinte de culture (1). L'air, de densité inférieure au liquide, monte rapidement sous forme de bulles. Le liquide et les microalgues sont entraînés par le mouvement ascensionnel des bulles. L'injection d'air peut-être effectuée de façon verticale mais également de façon oblique afin de provoquer des transports de liquide d'une paroi à l'autre du milieu de culture favorisant le mélange des nutriments et du CO₂ nécessaires aux microalgues. Ce mouvement du liquide de culture assure également un éclairement moyen à toutes les microalgues lors de leur montée Les microalgues redescendent alors dans les volumes ou il n'y a pas de remontée de bulles d'air. On a ainsi réalisé une boucle fermée de parcours du liquide de culture. Cette technique permet un mélange faiblement énergivore et faiblement stressant pour les microalgues.

Le brassage du milieu de culture peut être réalisé en partie par un système classique air-lift, qui donne essentiellement une impulsion verticale, complété par un système original d'injection latérale (CO₂ + air) distribuée à l'aide de « feeders » (14) sur la hauteur de l'enceinte de culture. On appelle ici « feeders » une canalisation ou un tube permettant le transport de gaz ou d'eau de la source jusqu'à l'endroit où l'on désire injecter le gaz ou l'eau. Les dits « feeders » (14) seront installés dans la zone de culture contre les parois (20) des enceintes étanches (5) ou de l'enceinte de culture (1). Les buses d'injection (15) sont distribuées sur un (ou des) « feeder(s) » (14). Leur nombre ainsi que leur inclinaison seront fonction du type d'impulsion que l'on souhaite transmettre aux microorganismes (impulsion transverse, impulsion verticale, ou impulsion permettant de donner un mouvement d'ensemble à la biomasse, qui permet aux algues d'aller périodiquement d'un bord à l'autre du réacteur, avec un mouvement ascensionnel). Avantageusement, cette capacité de gérer le mouvement transverse de la biomasse sera utilisée pour l'homogénéisation de l'éclairement de celle-ci, c'est-à-dire préférentiellement dirigée vers le haut avec une inclinaison précise. De plus, dans cette configuration de réacteur, il est possible d'adapter l'intensité de l'impulsion transverse de manière que le temps de transit des microorganismes entre les zones éclairées et non éclairées conduise à réaliser spatialement le cycle d'éclairement nécessaire à la croissance de certains types d'algues (temps d'éclairement/ temps d'extinction).

Avantageusement, on prélève régulièrement ou de façon continue en partie haute de l'enceinte de culture (1) un volume de culture qui est aussitôt remplacé par l'injection d'un volume équivalent d'eau contenant des éléments nutritifs en partie basse de l'enceinte de culture (1) ou dans les « feeder(s) » (14). Cette méthode permet de contribuer à diminuer l'énergie nécessaire pour induire la circulation du liquide dans le réacteur.

Le système de refroidissement (9) permet d'extraire la chaleur dégagée par les cellules photovoltaïques (2) tout en ajustant la température du milieu de culture (3) du photobioréacteur.

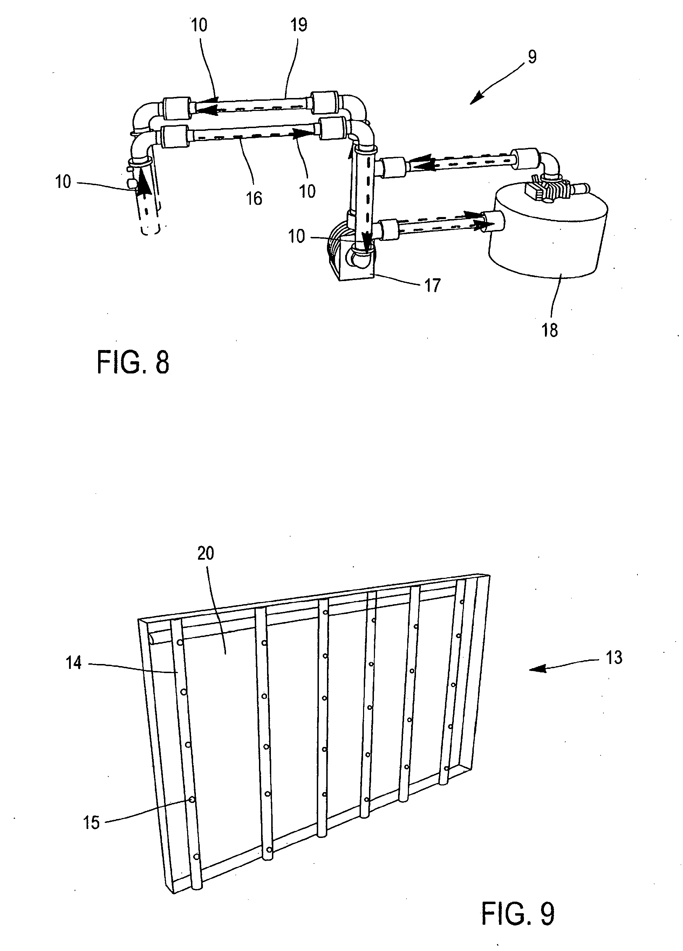

Le système de refroidissement (9) peut consister en un échangeur de chaleur. Par exemple, cet échangeur de chaleur consiste en des moyens pour acheminer (16) le liquide caloporteur (10) chaud vers l'extérieur de l'enceinte de culture (1), par exemple des tuyaux reliés à l'extrémité supérieure de l'enceinte de culture (1) couplés à une pompe (17), et un refroidisseur (18) consistant à faire circuler le liquide caloporteur chaud en sens inverse d'eau froide (voir Figure 8). Avantageusement, le liquide caloporteur (10) sort de l'enceinte de culture (1) à une de ses extrémités, en haut ou en bas et entre dans l'enceinte de culture (1) par l'autre extrémité. Le liquide caloporteur (10) froid retourne dans l'enceinte de culture (1) par des moyens pour l'acheminer (19), par exemple des tuyaux.

Avantageusement, le nombre de cellules photovoltaïques dans le photobioréacteur de l'invention est tel qu'elles recouvrent des plaques (7) qui s'étendent environ sur toute la hauteur de l'enceinte de culture (1).

La disposition des plaques (7) de cellules photovoltaïques (2) est adaptée à la forme du photobioréacteur.

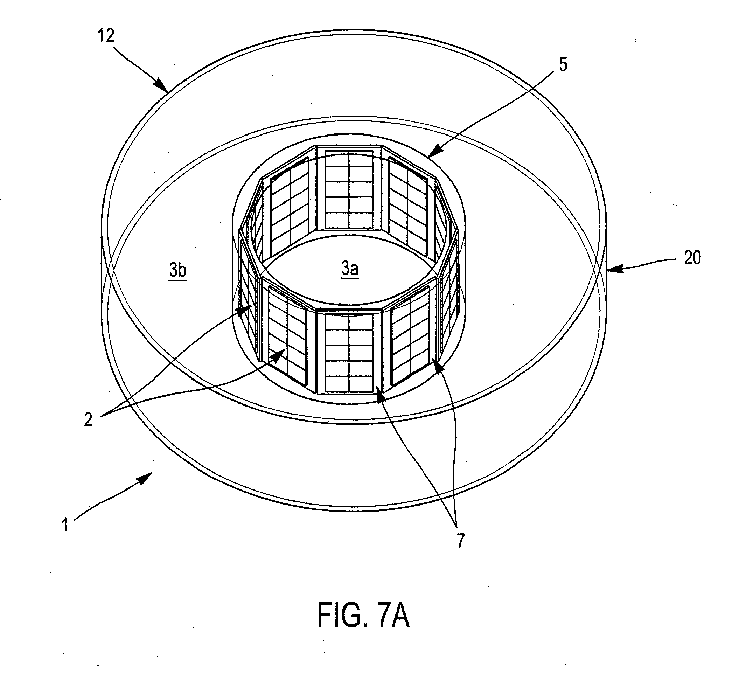

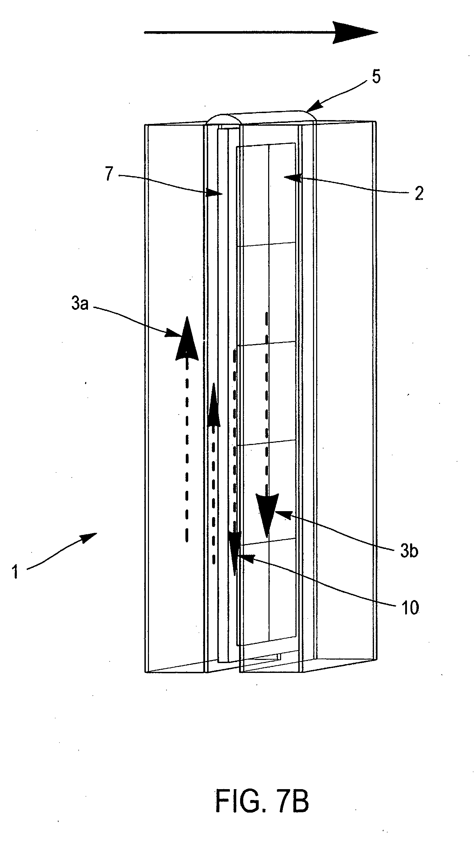

Par exemple, lorsque le photobioréacteur présente une enceinte de culture de forme cylindrique, les plaques forment entre elles un tube de section polygonale, de préférence hexagonale ou octogonale, pour approcher au mieux la forme cylindrique (voir Figures 7).

Afin de corriger les effets de bord dans les angles du polygone, l'intensité du courant d'injection pourra être adaptée localement puisque l'intensité lumineuse est proportionnelle à l'intensité du courant d'injection. L'intensité du courant d'injection peut être adaptée en modulant le pas du réseau de contacts d'injection (8).

Il est également possible d'utiliser un matériau diffusant polymère pour améliorer l'homogénéisation du front d'onde. Ce matériau en film mince peut recouvrir les parois externes (20) des enceintes étanches (5) lorsque les plaques (7) de cellules photovoltaïques sont placées dans l'enceinte de culture ou les parois de l'enceinte de culture (6) lorsque les plaques (7) de cellules photovoltaïques (2) sont placées à l'extérieur de l'enceinte de culture (1) à faible distance des parois externes (6).

Un autre objet de l'invention est l'utilisation de cellules photovoltaïques (2) alimentées électriquement en mode inverse d'émission de lumière pour éclairer le milieu de culture d'un photobioréacteur.

Un autre objet de l'invention est l'utilisation d'un photobioréacteur selon l'invention pour cultiver des micro-organismes photosynthétiques, de préférence des microalgues.

D'autres caractéristiques et avantages de l'invention apparaîtront mieux à la lecture de la description des modes de réalisation de l'invention. La description se réfère aux figures annexées suivantes.

### Figures

Figure 1 : Diagramme d'émission d'une LED
Figure 2 : Diagramme d'émission d'une cellule photovoltaïque
Figure 3 : Diagramme d'émission d'une cellule photovoltaïque avec renforcement des courants d'injection sur les bords
Figure 4 : Diagramme d'émission d'une juxtaposition de LEDs
Figure 5 : Diagramme d'émission d'un plan de cellules photovoltaïques juxtaposées
Figure 6a-6b : Schémas en perspective et de face d'un photobioréacteur parallélépipédique comprenant une plaque de cellules photovoltaïques qui s'insère entre deux enceintes de culture
Figure 7a-7b : Schémas en perspective et selon une section radiale d'un photobioréacteur cylindrique comprenant des cellules photovoltaïques disposées sur un tube de section hexagonale placées dans un tube étanche immergé dans le milieu de culture.
Figure 8: Présentation du système de refroidissement des cellules photovoltaïques et du système de régulation de la température du photobioréacteur
Figure 9 : Schéma d'un détail du système de brassage du milieu de culture installé sur une paroi.

Le photobioréacteur selon l'invention sera expliqué en référence aux Figures.

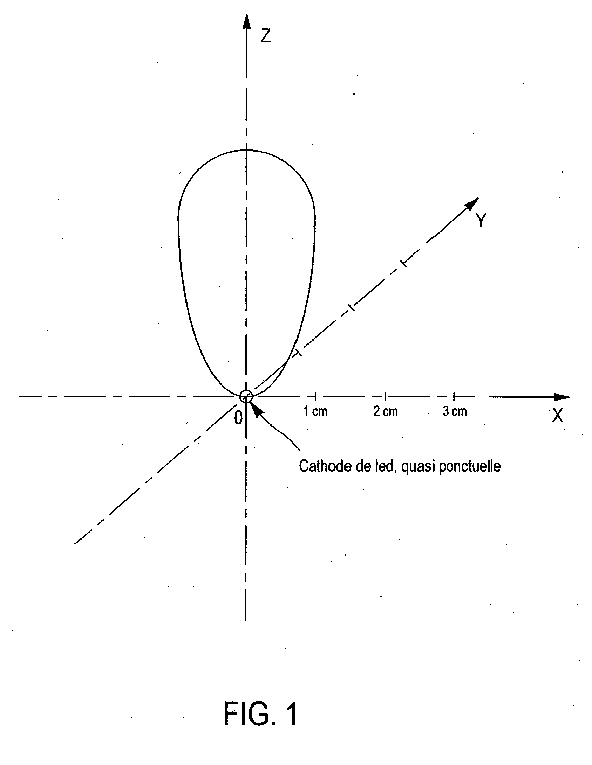

Les figures 1 à 5 sont des diagrammes d e l'émission énergétique. Une led quasi ponctuelle émet sont énergie de manière « Lambertienne » (lobe). L'essentiel de l'énergie étant émise perpendiculairement à la surface du semi-conducteur. Cette énergie décroit quand on s'écarte de la normale au semi-conducteur. Elle est nulle parallèlement à la surface de celui-ci. L'extension de la surface émissive au-delà de la largeur naturelle du lobe permet par sommation des lobes élémentaires de faire une surface émissive constante en énergie dans les plans parallèles à la surface du semiconducteur (xOy). Dans les figures, la LED ou la cellule photovoltaïque est centrée en O et sa surface est orientée perpendiculairement à (Oz). Une coupe de ces lobes est représentée selon le plan (xOz).

La figure 1 représente le diagramme d'émission d'une LED située au centre du repère. La cathode est supposée quasi-ponctuelle (d'une taille inférieure au mm²). Il y a invariance par rotation autour de l'axe (Oz).

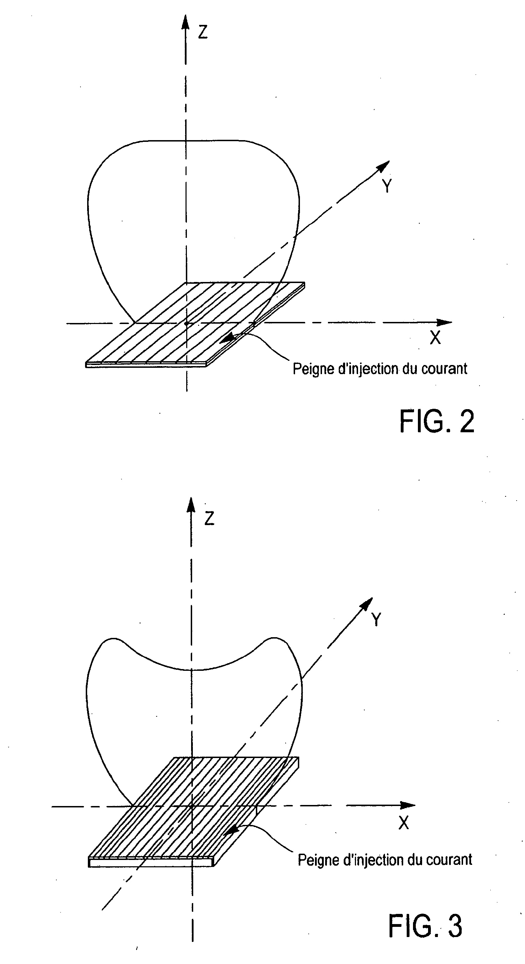

La figure 2 représente le diagramme d'émission d'une cellule photovoltaïque inversée comme utilisée par l'invention, ici avec un espacement constant des doigts d'injection du courant. L'intensité lumineuse dans un plan parallèle à (xOy) est constante au voisinage du centre de la cellule.

La figure 3 représente le diagramme d'émission énergétique d'une cellule photovoltaïque inversée dans le cas d'un resserrement de l'espacement des doigts d'injection du courant en approchant des bords. La densité de courant injecté est plus importante sur les bords, d'où l'augmentation de l'intensité lumineuse.

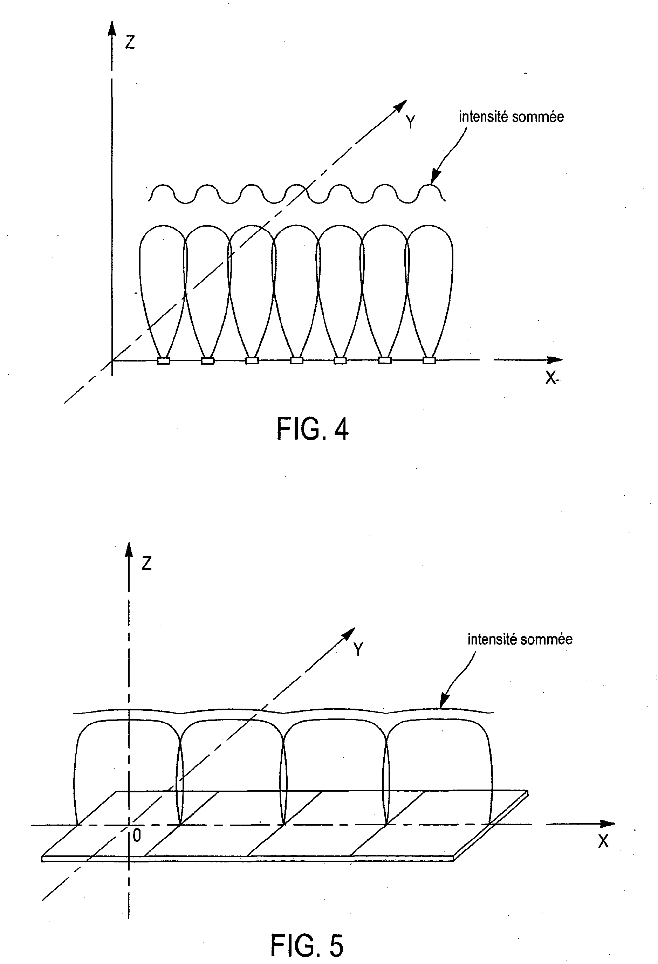

La figure 4 représente le diagramme d'émission d'une barrette de LEDs (disposées suivant (Ox)). La sommation des flux lumineux conduit à un front inhomogène, dont l'inhomogénéité dépend de la distance entre deux LEDs consécutives sur la barrette.

La figure 5 représente le diagramme d'émission d'une barrette de cellules (disposées suivant (Ox)). Si les cellules sont suffisamment proches, l'intensité lumineuse dans un plan parallèle à (xOy) est constante, l'énergie reçue ne dépend donc plus que de la distance à la cellule : en effet l'homogénéité du flux est indépendante de la distance à laquelle est faite la mesure.

Selon un premier mode de réalisation, le photobioréacteur est cylindrique (Figures 7). Des cellules photovoltaïques (2) sont disposées sur les deux faces de six plaques (7) formant entre elles un tube de section hexagonale. Ces plaques (7) ont pour longueur la hauteur du photobioréacteur. Ces plaques (7) sont placées dans un tube étanche (5) en matériau transparent à la lumière (verre plastique..), lui-même immergé dans le milieu de culture (3), qu'il sépare entre une partie « interne » (3a), et une partie « externe » (3b), visibles Figure 7a. Les plaques sont reliées à des contacts d'injection de courant (8).

Selon un deuxième mode de réalisation, le photobioréacteur est parallélépipédique (Figures 6). Des cellules photovoltaïques (2) sont disposées sur les deux faces d'une ou plusieurs plaques (7) métalliques. Ces plaques ont pour dimensions celles du photobioréacteur. Ces plaques (X) sont placées à l'extérieur du photobioréacteur, de préférence entre deux enceintes de culture empilées. Les plaques sont reliées à des contacts d'injection de courant (8). Les cellules photovoltaïques sont isolées électriquement de la plaque métallique par un isolant de bonne conductivité thermique de type Mylar®.

## Revendications

1. Photobioréacteur destiné à la culture de micro-organismes photosynthétiques, de préférence de microalgues, comprenant :
(a) au moins une enceinte de culture (1) destinée à contenir le milieu de culture (3) des microorganismes,
(b) des cellules photovoltaïques (2) isolées du milieu de culture (3) émettant de la lumière vers le milieu de culture (3)
(c) des moyens d'alimentation électrique (4) des cellules photovoltaïques (2) afin de faire fonctionner les cellules photovoltaïques en mode d'émission de lumière.

2. Photobioréacteur selon la revendication 1, **caractérisé en ce que** les cellules photovoltaïques (2) sont disposées sur des plaques, préférentiellement en en recouvrant toute la surface.

3. Photobioréacteur selon la revendication 1 ou 2, **caractérisé en ce que** les cellules photovoltaïques (2) sont des cellules à une ou deux jonctions.

4. Photobioréacteur selon l'une quelconque des revendications précédentes, **caractérisé en ce que** les cellules photovoltaïques (2) sont en matériau à gap direct III/V.

5. Photobioréacteur selon l'une quelconque des revendications précédentes, **caractérisé en ce que** les cellules photovoltaïques (2) sont placées dans des enceintes de transparence adaptée-(TA) et étanches (5) immergées dans le milieu de culture (3).

6. Photobioréacteur selon l'une quelconque des revendications 1 à 4, **caractérisé en ce que** les cellules photovoltaïques (2) sont placées à l'extérieur de la ou des enceinte(s) de culture à faible distance de la paroi externe de la ou des enceintes de culture et la paroi externe de la ou des enceinte(s) de culture est constituée d'un matériau de transparence adaptée au passage de la ou des longueurs d'ondes qu'émettent lesdites cellules photovoltaïques.

7. Photobioréacteur selon la revendication 6, comprenant plusieurs enceintes de culture parallélépipédiques empilées et séparées par des plaques (7) de cellules photovoltaïques (2).

8. Photobioréacteur selon l'une quelconque des revendications précédentes, comprenant un système de refroidissement (9) des cellules photovoltaïques (2).

9. Photobioréacteur selon l'une quelconque des revendications précédentes, comprenant un système de brassage (13) du milieu de culture (3).

10. Photobioréacteur selon les revendications 1 à 5 et 8 à 9, comprenant :
(a) une enceinte de culture (1) cylindrique destinée à contenir le milieu de culture (3) des microorganismes,
(b) des cellules photovoltaïques (2) isolées du milieu de culture (3) recouvrant des plaques (7), lesdites plaques s'étendant sur environ toute la hauteur de l'enceinte de culture (1), placées dans un tube de transparence adaptée et étanche (5) immergé dans le milieu de culture (3) et disposées en un tube de section polygonale.

11. Photobioréacteur selon les revendications 1 à 4 et 6 à 9, comprenant :
(a) plusieurs enceintes de culture (1) parallélépipédiques empilées et séparées par
(b) des plaques (7) de cellules photovoltaïques (2), les dites plaques présentant les dimensions d'une face d'enceinte de culture.

12. Utilisation de cellules photovoltaïques alimentées électriquement en mode inverse d'émission de lumière pour éclairer le milieu de culture d'un photobioréacteur.

13. Utilisation d'un photobioréacteur selon l'une quelconque des revendications 1 à 11, pour cultiver des micro-organismes photosynthétiques, de préférence des microalgues.

## Patentansprüche

1. Fotobioreaktor zur Kultivierung von fotosynthetischen Mikroorganismen, vorzugsweise Mikroalgen, umfassend:
(a) Wenigstens einen Kulturbehälter (1), dazu bestimmt das Kulturmedium (3) der Mikroorganismen zu enthalten,
(b) Fotovoltaikzellen (2), getrennt von dem Kulturmedium (3) (und) Licht in Richtung des Kulturmediums abgebend,
(c) Elektrische Versorgungsmittel (4) der Fotovoltaikzellen (2) um die Fotovoltaikzellen im Lichtabgabemodus zu betreiben.

2. Fotobioreaktor gemäß Anspruch 1, **dadurch gekennzeichnet, dass** die Fotovoltaikzellen (2) auf Platten angeordnet sind, vorzugsweise deren ganze Oberfläche bedeckend.

3. Fotobioreaktor gemäß Anspruch 1 oder 2, **dadurch gekennzeichnet, dass** die Fotovoltaikzellen (2) Zellen mit einem oder zwei Übergängen sind.

4. Fotobioreaktor gemäß einem der vorstehenden Ansprüche, **dadurch gekennzeichnet dass** die Fotovoltaikzellen (2) aus Materialien mit direkter III/V Bandlücke sind.

5. Fotobioreaktor gemäß einem der vorstehenden Ansprüche, **dadurch gekennzeichnet, dass** die Fotovoltaikzellen (2) in wasserdichten Behältern (5) mit angepasster Transparenz (TA) angeordnet sind, welche in dem Kulturmedium (3) eingetaucht sind.

6. Fotobioreaktor gemäß einem der Ansprüche 1 bis 4, **dadurch gekennzeichnet dass** die Fotovoltaikzellen (2) außerhalb des oder der Kulturbehälter in geringem Abstand von der Außenwand des oder der Kulturbehälter angeordnet sind und dass die Außenwand des oder der Kulturbehälter aus einem Material besteht, dessen Transparenz an das Durchlassen der durch die Fotovoltaikzellen abgegebenen Wellenlänge(n) angepasst ist.

7. Fotobioreaktor gemäß dem Anspruch 6, umfassend mehrere gestapelte quaderförmige Kulturbehälter, welche durch Platten (7) aus Fotovoltaikzellen (2) getrennt sind.

8. Fotobioreaktor gemäß einem der vorstehenden Ansprüche, umfassend ein Kühlsystem (9) der Fotovoltaikzellen (2).

9. Fotobioreaktor gemäß einem der vorstehenden Ansprüche, umfassend ein Rührsystem (13) des Kulturmediums (3).

10. Fotobioreaktor gemäß einem der Ansprüche 1 bis 5 und 8 bis 9, umfassend:
(a) Einen zylindrischen Kulturbehälter (1), dazu bestimmt, das Kulturmedium (3) der Mikroorganismen zu enthalten,
(b) Fotovoltaikzellen (2), getrennt von dem Kulturmedium (3), welche Platten (7) bedecken, wobei die Platten sich in etwa über die Höhe des Kulturbehälters (1) erstrecken, in einem wasserdichten Rohr (5) mit angepasster Transparenz angebracht sind und in einem Rohr mit polygonalem Querschnitt angeordnet sind.

11. Fotobioreaktor gemäß einem der Ansprüche 1 bis 4 und 6 bis 9, umfassend:
(a) Mehrere gestapelte, quaderförmige Kulturbehälter (1), getrennt durch
(b) Platten (7) aus Fotovoltaikzellen (2), wobei die Platten die Maße einer Kulturbehälterseite aufweisen.

12. Verwendung von elektrisch versorgten Fotovoltaikzellen im invertierten Modus zur Lichtabgabe um das Kulturmedium eines Fotobioreaktors zu erhellen.

13. Verwendung eines Fotobioreaktors gemäß einem der Ansprüche 1 bis 11, um fotosynthetische Mikroorganismen, vorzugsweise Mikroalgen, zu kultivieren.

## Claims

1. Photobioreactor for cultivating photosynthetic micro-organisms, preferably microalgae, comprising:
(a) at least one culture enclosure (1) for containing the culture medium (3) of the micro-organisms,
(b) photovoltaic cells (2) isolated from the culture medium (3) emitting light to the culture medium (3)
(c) means (4) for supplying electrical power to the photovoltaic cells (2) in order to operate the photovoltaic cells in light emission mode.

2. Photobioreactor according to claim 1, **characterised in that** the photovoltaic cells (2) are arranged on panels, preferentially covering the entire surface.

3. Photobioreactor according to claim 1 or 2, **characterised in that** the photovoltaic cells (2) are cells with one or two junctions.

4. Photobioreactor according to any of the preceding claims, **characterised in that** the photovoltaic cells (2) are made of a III/V direct gap material.

5. Photobioreactor according to any of the preceding claims, **characterised in that** the photovoltaic cells (2) are placed in sealed containers (5) of adapted transparency (TA) immersed in the culture medium (3).

6. Photobioreactor according to any of claims 1 to 4, **characterised in that** the photovoltaic cells (2) are placed outside the culture enclosure(s), at a short distance from the external wall of the culture enclosure(s) and the external wall of the culture enclosure(s) consists of a material of adapted transparency for the passage of the wavelength(s) emitted by said photovoltaic cells.

7. Photobioreactor according to claim 6, comprising a plurality of parallelepipedic culture enclosures, stacked and separated by panels (7) of photovoltaic cells (2).

8. Photobioreactor according to any of the preceding claims, comprising a system (9) for cooling the photovoltaic cells (2).

9. Photobioreactor according to any of the preceding claims, comprising a system (13) for mixing the culture medium (3).

10. Photobioreactor according to claims 1 to 5 and 8 to 9, comprising:
(a) a cylindrical culture enclosure (1) for containing the micro-organism culture medium (3),
(b) photovoltaic cells (2) isolated from the culture medium (3) covering panels (7), said panels extending along approximately the entire height of the culture enclosure (1), placed in a sealed tube (5) of adapted transparency immersed in the culture medium (3) and arranged in a tube having a polygonal cross-section.

11. Photobioreactor according to claims 1 to 4 and 6 to 9, comprising:
(a) a plurality of parallelepipedic culture enclosures (1), stacked and separated by
(b) panels (7) of photovoltaic cells (2), said panels having the dimensions of one face of the culture enclosure.

12. Use of electrically powered photovoltaic cells in inverted light emission mode to illuminate the culture medium of a photobioreactor.

13. Use of a photobioreactor according to any of claims 1 to 11, for cultivating photosynthetic micro-organisms, preferably microalgae.
